Europäisches Patentamt

European Patent Office

Office européen des brevets

(11) Publication number: **0 257 472**
**A2**

# EUROPEAN PATENT APPLICATION

(21) Application number: **87111760.2**

(22) Date of filing: **13.08.87**

(51) Int. Cl.⁴: **C12N 15/00 , A01H 1/00 , A01H 5/10**

(30) Priority: **14.08.86 EP 86111291**

(43) Date of publication of application:
**02.03.88 Bulletin 88/09**

(84) Designated Contracting States:
**AT BE CH DE ES FR GB GR IT LI LU NL SE**

(71) Applicant: **Max-Planck-Gesellschaft zur Förderung der Wissenschaften e.V.
Bunsenstrasse 10
D-3400 Göttingen(DE)**

(72) Inventor: **De La Pena, Alicia, Dr.
Ostlandstrasse 49
D-5000 Köln 40(DE)**
Inventor: **Lörz, Horst, Dr.
Goldammer Weg 3
D-5000 Köln 30(DE)**
Inventor: **St. Schell, Jozef, Prof. Dr.
c/o Max Planck-Institut für Züchtungsforschung
Egelspfad D-5000 Köln 30 (Vogelsang)(DE)**

(74) Representative: **Vossius & Partner
Siebertstrasse 4 P.O. Box 86 07 67
D-8000 München 86(DE)**

(54) **Transgenic monocotyledonous plants, seeds thereof and process for the preparation of the plants.**

(57) Described are transgenic monocotyledonous plants, in particular transgenic cereal plants containing at least one foreign DNA segment of interest integrated in their genome. Furthermore, seeds of said transgenic monocotyledonous plants are described. Additionally, a process for preparing said transgenic monocotyledonous plants is provided.

EP 0 257 472 A2

## "Transgenic Monocotyledonous Plants, Seeds thereof and Process for the Preparation of the Plants"

### Technical Field of the Invention

The invention relates to transgenic monocotyledonous plants, in particular transgenic cereal plants containing at least one foreign DNA segment of interest integrated in their genome. The invention furthermore relates to seeds of said transgenic monocotyledonous plants, in particular transgenic cereal plants. Additionally, the invention relates to a process for preparing said transgenic monocotyledonous plants, in particular transgenic cereal plants.

### Background Art

To date, the most successful method to produce transgenic plants has been based on the ability of Agrobacterium tumefaciens to infect numerous higher plant species and to transfer a defined DNA fragment (T-DNA) to the genome of the infected cells (see e.g. EP-A-0 116 718). However, one major limitation of this procedure is the fact that the agriculturally important cereals, such as rye, wheat, spelt, barley, oat, millet, rice, sugar cane, and maize cannot readily be transformed with Agrobacterium.

The direct gene transfer into a protoplast has shown that purified exogenous DNA can be taken up, integrated and expressed in cells of a variety of plant species including some cereals. However, these experiments involved the use of protoplasts. Although it is possible to grow protoplasts into unorganized tissue (calli), it has so far only been possible to regenerate fully organized mature rice plants from protoplast-derived tissue.

### Brief Summary of the Invention

It is the object of the invention to provide agriculturally important transgenic monocotyledonous plants, in particular transgenic cereal plants, e.g. plants belonging to the family of Gramineae, such as rye, wheat, spelt, barley, oat, millet, rice, sugar cane or maize, which contain at least one foreign DNA segment of interest integrated in their genome.

Furthermore, it is an object of the present invention to provide the seeds of said transgenic plants which are useful for the continuous production of further generations of said plants.

It is another object of the present invention to provide a convenient and reliable process for producing said plants, which dispenses with the preparation of protoplasts, thus avoiding the laborious regeneration of plants from the protoplasts.

### Detailed Description of the Invention

In a previous publication, the cytological analysis of the development of the male germ line of rye (Secale cereale L.) has shown that about 14 days prior to the first meiotic metaphase, the archesporial cells are highly permeable to caffeine and colchicine which is injected into the young immature ears (De la Pena et al., Chromosoma 83 (1981), 241-248; Puertas et al., Chromosoma 89 (1984), 121-126).

Surprisingly it has now been found that during premeiotic developmental stages cells of monocotyledonous plants are permeable not only to small molecules, such as the above-mentioned caffeine and colchicine, but also to macromolecules, such as DNA molecules.

In the process of the present invention, a sufficient amount of a vector containing a foreign DNA segment of interest which is under the control of transcription sequences allowing the expression of the foreign DNA in the plant is injected into at least one of the young immature ears of a monocotyledonous plant. Preferably, the plant is kept in a dry environment without being watered about one day before and two days after the injection to enhance the absorption of the infected DNA.

The DNA to be incorporated into the plant cells after the injection may be any vector, such as a plasmid or a cosmid, containing a foreign DNA segment of interest, irrespective of whether or not the foreign DNA segment itself carries a selectable marker gene. The only important point is that the incorporation of the foreign DNA can be detected by biochemical, molecular or genetic analysis.

Examples of such a foreign DNA segment are segments encoding antibiotic resistance, herbicide resistance, particular colours, or the resistance to diseases caused for instance by insects, fungi, bacteria or viruses.

Transcription promoting sequences allowing the expression of a foreign DNA in a plant cell are known in the art.

After the injection of the vector, the young immature ears are allowed to grow to maturity, then pollination is effected. When the plant is self-compatible, self-pollination is performed. If the plant is self-incompatible, injected mature ears obtained from different plants are pair-wise crossed.

Subsequently, the plant is allowed to grow until the seeds are ripe. Then the seeds are screened to identify those seeds which carry the foreign DNA segment of interest.

If the new genotype is not characterized by readily recognizable phenotypic parameters, the seeds obtained are germinated and the growing seedlings are screened for the expression of the vector DNA and/or the foreign DA segment.

The subsequent screening is carried out according to known methods. In the case of a transferred antibiotic or herbicide resistance, for instance, the seedlings are cultivated in the presence of the substance at a concentration that is lethal to control seedlings, in the case of a transferred colour gene, the colour of the particular structure of the plant is evaluated, or in the case of disease resistance, the seedlings are infected with the pathogenic agent that is lethal to the control seedlings.

The screening can also be carried out according to the well known Southern, Northern or Western Blot procedures. Furthermore, a specific assay can be performed in which the biological activity of the protein encoded by the transferred gene is determined.

Seedlings which gave a positive result in the screening assay are cultivated until the seeds are ripe. The harvested seeds can be propagated to obtain further generations of the transgenic plant.

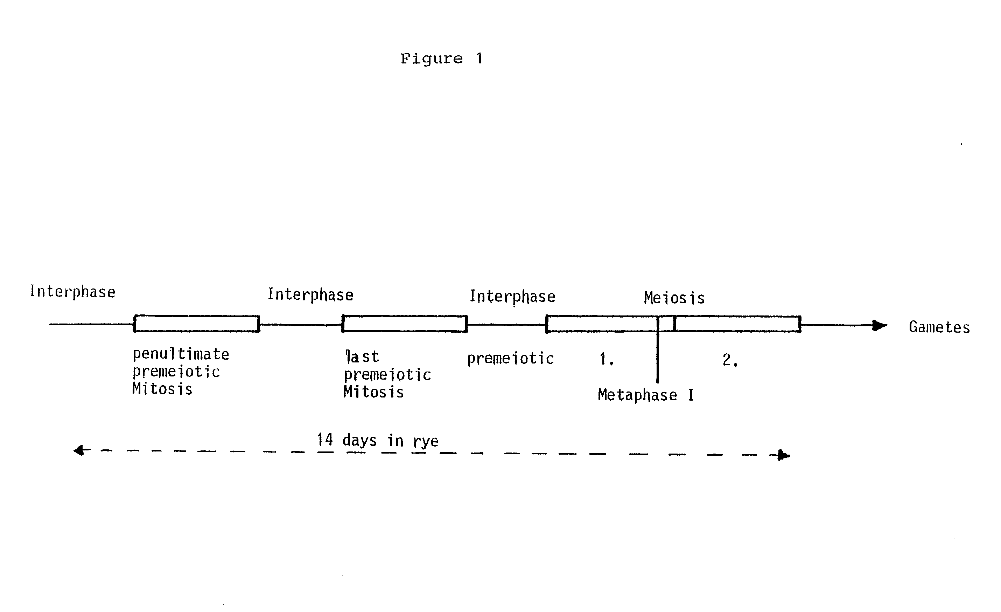

In the process of the present invention it is preferred to inject the DNA during the first interphase before the penultimate premeiotic mitosis (see Figure 1). This interphase can be generally recognized by the following procedure:
-localization of the meiosis metaphase I according to known cytological methods in the region of the middle spikelet of the young immature ear;
-determination of the number of PMC's (P ollen Mother Cells) per pollinic sac, e.g. according to the method of Puertas et al. (loc.cit.) or by conventional counting;
-identification of younger developmental stages in which the number of cells per pollinic sac is a quarter of the number of cells in metaphase I; and
-identification of mitotic divisions in the surroundings of the identified developmental stage.

The above procedure is suitable for the determination of the interphase before the penultimate premeiotic mitosis of female and male flowers.

In a preferred embodiment of the present process, the injection is given to each internode underneath the young immature ear.

In another preferred embodiment, the injection is performed by an injection means, such as a needle with a small diameter or a glass capillary with or without a micromanipulator.

In another preferred embodiment, the DNA is suspended in an aqueous hypotonic medium before being injected. Hypontonic media promote the absorption of the DNA by the plant.

It is also preferred to use a DNA containing medium having a concentration of about 10 to 100 $\mu$g/ml of vector DNA.

Finally, in the process of the present invention the plant is preferably injected with 10 to 1,000 $\mu$l of the DNA-containing medium. Most preferred are 10 to 300 $\mu$l in the case of small plants and 100 to 1,000 $\mu$l in the case of large plants.

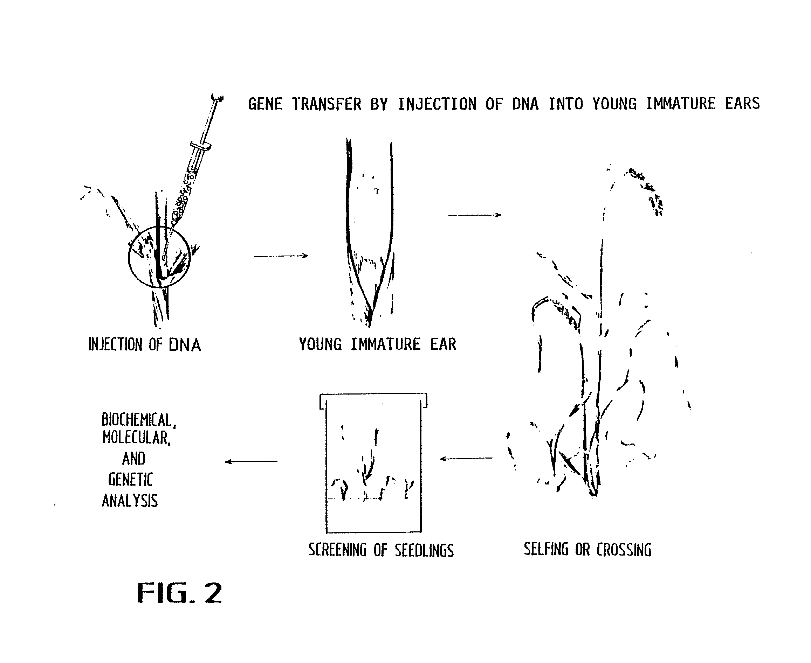

The process of the present invention is - schematically outlined in Fig. 2.

According to the process of the present invention, transgenic monocotyledonous plants are provided which contain at least one foreign DNA segment of interest integrated in their genome. Preferably, transgenic monocotyledonous plants of the family Gramineae, such as spelt, oat, millet, rice, sugar cane, and maize are provided according to the present invention. More preferably, transgenic monocotyledonous plants of the family Granineae, such as barley and wheat, and most preferably, transgenic rye is provided by the present invention.

The rye system is considered a useful model for monocotyledonous plants which have a premeiotic development equivalent to that of rye.

Brief Description of the Drawings

Fig. 1 shows a timing schedule of the premeiotic developmental stages of monocotyledonous plants.

Fig. 2 shows a scheme of the transformation and screening strategy.

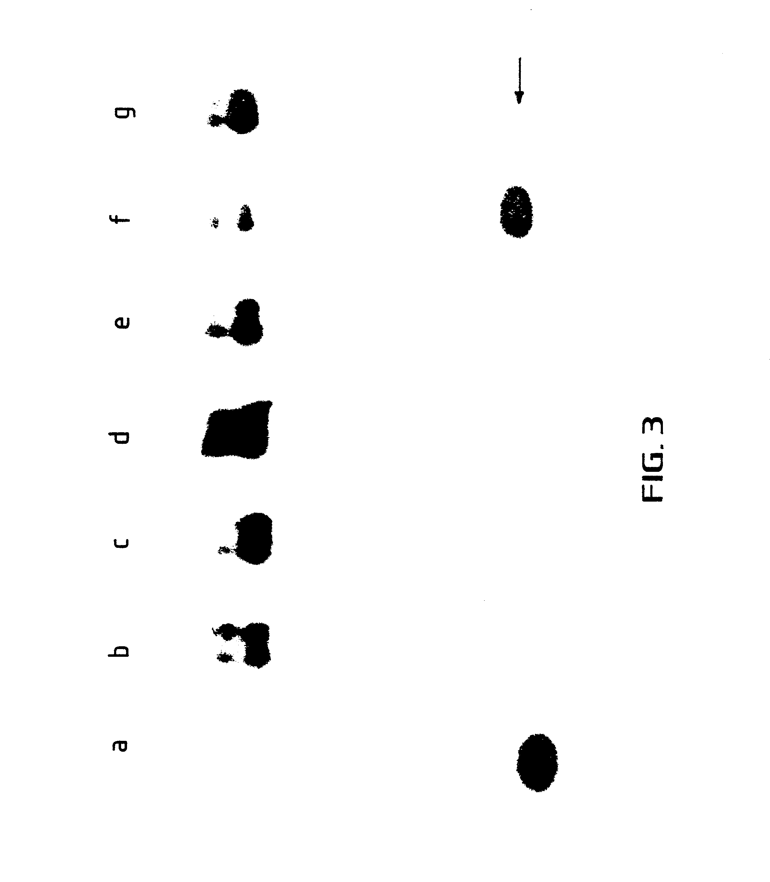

Fig. 3 shows the result of an assay for APH (3')II activity in plant extracts. About 100mg of leaf material were crushed in an Eppendorf tube with 70 $\mu$l of extraction buffer (Schreier et al., EMBO J4 (1985),25-32). 50 $\mu$l of each protein extract were subjected to electrophoresis in a 10% polyacrylamide gel. The gel was incubated with kanamycin and gamma$^{32}$P-labelled ATP and blotted onto Whatman phosphocellulose paper p81. An X-ray film was exposed to the blot for 1 to 2 days at -70°C with an intensifying screen. Lane a: APH-(3') II transformed tobacco (positive control). Lane b: Untreated JNK rye (negative control). Lanes c to e: untransformed,kanamycin-sensitive seedlings from injected plants. Lane f: kanamycin-resistant seedlings. Lane g: apparently kanamycin-resistant seedlings showing no APH(3')II activity. The arrow shows the band of kanamycin³²phosphate which comigrates with the resulting from the APH(3')II protein of a transformed tobacco plant.

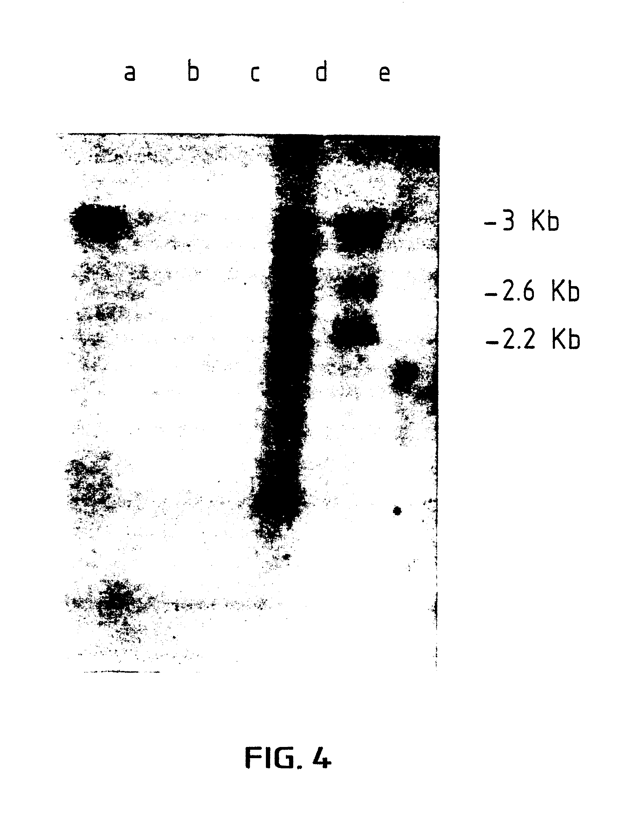

Fig. 4 shows an analysis of the DNA of an APH(3')II positive plant. The DNA was isolated from leaf material and subjected to double digestion with the restriction endonucleases EcoRI and HindIII. Approximately 5 μg of digested DNA were analysed by electrophoresis in an 0.8% agarose gel. Subsequently, a Southern blot was prepared. The blot was hybridized with a labelled 1.6 kb internal PstI-fragment of the APH(3')II gene. Lane a: pLGVneo1103 digested with EcoRI and HindIII. Lane b: JNK rye nontreated, digestion with EcoRI and HindIII. Lane c: DNA from a normal control rye plant, non-injected. Lane d: DNA from an APT(3')II positive plant, Lane e: DNA from an APH(3')II positive plant. As indicated, the upper band corresponds to a DNA fragment having a length of 3kb. This fragment comigrates with the EcoRI/HindIII fragment of plasmid pLGVneo1103. Two additional bands corresponding to DNA fragments of 2.6 and 2.2kb hybridized with the probe. Most probably, they are the result of rearrangements of the gene introduced.

The following examples illustrate the invention.

## Example 1

### Preparation of a transgenic rye plant

300 μl of a 1,000 μg/ml aqueous suspension of the plasmid pLGVneo1103 carrying the aminoglycosidephosphotransferase II gene (APH-(3')II) under the control of the nopaline synthase promotor (pNOS) were injected underneath the young immature ears of the diploid rye cultivar JNK. The injection was stopped when several drops of the suspension emerged from the top of the ears. The plants were injected 14 days before meiosis as shown in the scheme in Fig. 2. In rye, this stage can be easily recognized by morphological parameters of the young ears (De la Pena et al., loc. cit.). The young ears already have 5 leaves and between 1/2 and 1/3 of the flag leaf can be seen. The young ear is approximately 2 cm long and can be detected at about the level of the third leaf. A tuberculine syringe (25G 5/8) was used. After the injection, the young immature ears were allowed to grow until reaching maturity and subsequently, they were crossed among themselves. Self-pollination was not effected, since the JNK rye is highly self-incompatible. The seeds harvested from the injected ears were screened for kanamycin resistance. 3 l glass containers were filled under aseptic conditions with 2 ml of Knop 1 865 nutrition solution supplemented with 10 mg/l of kanamycin sulfate (Merck). The seeds were surface sterilized and allowed to germinate while laying on a plastic net located inside the glass container just above the liquid level. A maximum of 10 seeds were tested in each container located in a culture chamber at 26°C and 2,000 lux for 16 hours/day. Control seeds from non-treated rye plants were germinated under the same conditions with and without kanamycin. In the presence of kanamycin, the control seedlings started to bleach. After 10 days they were almost totally white. A total of 3,023 seeds derived from 98 plants injected with pLGVneo1103 were tested according to this procedure. Seven plants which remained green after 10 days were selected. These "kanamycin resistant" plants were assayed for the presence of APH(3')II enzymatic activity. The assay was performed according to the method of Reiss et al. (Gene 30 (1984), 217-223) modified for plant tissue according to Schreier et al. (loc. cit.). Two of the seven "kanamycin resistant" plants obtained from independent injection experiments, showed APH(3')II activity (see Fig.3). No APH(3')II activity was detected in about 100 control rye seedlings or in the kanamycin sensitive (bleached) seedlings derived from injected tillers.

To confirm that the observed APH(3')II activity was correlated with the presence of a pGLVneo1103 introduced into the plant genome, a Southern blot analysis of DNA isolated from both APH(3')II positive plants as well as from one APH-(3')II negative "kanamycin resistant" plant was performed (Southern et al., J.Mol.Biol. 98 (1975), 503-517). High molecular weight DNA was isolated from leaf tissue, essentially following the procedure of Dellaporta et al. (Plant Mol.Biol.Rep. 1 (1983), 19-21). A 1.6kb PstI fragment spanning the APH(3')II gene was used as a specific probe. The results of these experiments are shown in Fig. 4.

The results show that is possible for the first time to introduce genetic information into rye gametes and to recover the descendents in which the foreing gene is present and expressed. Most probably, the injected DNA is transported by the vascular system of the plant to the germ cells which can incorporate the DNA if they are in a "competent" stage. In our rye cultivar, this particular stage which presents itself two weeks before meiosis corresponds cytologically to the interphase previous to the penultimate premeiotic mitosis in the male side (Puertas et al., loc.cit.). During this stage, the cells undergo several physiological changes in response to the meiotic commitment.

The most important of these changes in relation to DNA up-take probably is the establishment of cytoplasmic channels connecting the archesporial cells.

## Example 2

### Preparation of a transgenic barley plant

The process described in Example 1 is repeated with the exception that young immature ears of barley were used instead of those of rye.

The obtained transgenic barley plants showed essentially the same resistance as described in Example 1.

## Example 3

### Preparation of a transgenic wheat plant

The process described in Example 1 was repeated with the exception that young immature ears of wheat were used instead of those of rye.

The obtained transgenic wheat plants showed essentially the same resistance as described in Example 1.

## Claims

1. Transgenic monocotyledonous plant containing at least one foreign DNA segment of interest integrated in its genome.

2. Transgenic plant according to claim 1, wherein the plant belongs to the family of Gramineae.

3. Transgenic plant according to claim 2, wherein the plant is rye.

4. Transgenic plant according to claim 2, wherein the plant is wheat.

5. Transgenic plant according to claim 2, wherein the plant is spelt.

6. Transgenic plant according to claim 2, wherein the plant is barley.

7. Transgenic plant according to claim 2, wherein the plant is oat.

8. Transgenic plant according to claim 2, wherein the plant is millet.

9. Transgenic plant according to claim 2, wherein the plant is rice.

10 Transgenic plant according to claim 2, wherein the plant is sugar cane.

11. Transgenic plant according to claim 2, wherein the plant is maize.

12. Seeds from transgenic monocotyledonous plants according to claim 1 or 2.

13. A process for preparing transgenic monocotyledonous plants according to claim 1, comprising injecting underneath at least one of the young immature ears of a monocotyledonous plant a sufficient amount of a vector containing a foreign DNA segment of interest which is under the control of transcription sequences allowing the expression of the foreign DNA in the plant, permitting the young immature ears to grow to maturity, performing pollination, allowing the plant to grow until the sees are ripe and then screening them.

14. A process for preparing transgenic monocotyledonous plants according to claim 1, comprising injecting underneath at least one of the young immature ears of a monocotyledonous plant a sufficient amount of a vector containing a foreign DNA segment of interest which is under the control of transcription promoting sequences allowing the expression of the foreign DNA in the plant, permitting the young immature ears to grow to maturity, performing pollination, allowing the plant to grow until the seeds are ripe, harvesting the seeds, permitting the seeds to germinate and then screening them for the expression of the vector DNA and/or the foreign DNA segment.

15. The process according to claim 13 or 14, wherein the injection is given during the first interphase before the penultimate premeiotic mitosis.

16. The process according to claim 13 or 14, wherein the injection is given to each internode underneath the young immature ears.

17. The process according to claim 13 or 14, comprising using as an injection means a needle with a small diameter or a glass capillary with or without a micromanipulator.

18. The process according to any one of claims 13 to 17, comprising injecting the vector DNA contained in a hypotonic aqueous medium

19. The process according to claim 18, comprising using 10 to 100 $\mu$g/ml vector DNA.

20. The process according to claim 18 or 19, comprising injecting 10 to 1,000 $\mu$l of the vector-DNA-containing medium.

21. The process according to any one of claims 13 to 20, wherein the plant is kept in a dry environment without being watered for about 1 day before and 2 days after the injection.

Figure 1

0 257 472

GENE TRANSFER BY INJECTION OF DNA INTO YOUNG IMMATURE EARS

INJECTION OF DNA

YOUNG IMMATURE EAR

BIOCHEMICAL,
MOLECULAR,
AND
GENETIC
ANALYSIS

SCREENING OF SEEDLINGS

SELFING OR CROSSING

FIG. 2

0 257 472

FIG. 3

a b c d e

−3 Kb

−2.6 Kb

−2.2 Kb

FIG. 4